# EUROPEAN PATENT SPECIFICATION

(11) **EP 3 640 380 B1**
(45) Date of publication and mention of the grant of the patent: **20.12.2023**
(21) Application number: 18746313.8
(22) Date of filing: 07.06.2018
(51) Int. Cl.: D03D 1/00, H01R 12/51, H01R 12/59, A61B 5/00, A61B 5/11, G08B 21/04

(54) **MONITORING DEVICE WITH OPTIMISED ELECTRICAL CONNECTIONS**
ÜBERWACHUNGSGERÄT MIT OPTIMISIERTEN ELEKTRISCHEN VERBINDUNGEN
AGENCEMENT DE SURVEILLANCE AVEC LIAISONS ÉLECTRIQUES OPTIMISÉES

(30) Priority: 13.06.2017 PT 2017110144
(43) Date of publication of application: 22.04.2020
(73) Proprietor: Flexitex - Fabrica De Tecidos SA, 3700-257 São João Da Madeira (PT)
(72) Inventor: DA S. RODRIGUES ANDRADE, Antonio Jose, 3800-735 Aveiro (PT); OLIVEIRA FREITAS, Diana Rosa, 4520-154 Santa Maria Da Feira (PT); RODRIGUES CHAVES FERREIRA, Paulo Alexandre, 1000-138 Lisboa (PT); GUIMARAES PIMENTA, Nelson Rafael, 1000-138 Lisboa (PT)
(74) Representative: Liebl, Thomas
(86) International application number: PCT/PT2018/000012
(87) International publication number: WO 2018/231081

(56) References cited:
- US-A1- 2004 266 244
- US-A1- 2012 156 926
- US-A1- 2017 042 340

## Description

The present invention refers to the field of cover dispositions for objects such as for example beds, and recognition of the presence and movement of individuals upon a respective support surface.

The present invention further refers to a monitoring system according to the present invention.

There are known several documents in prior art relating to dispositions for detecting movement of people in beds, including as monitoring means for application in hospitals and premises for accommodating senior individuals.

In particular, it is known using a disposition of the type pressure-operated electrical switch. Typically, said electrical switch is provided by the superposition of two conductors that result in contact when pressured together by the weight of an individual.

Documents US 4,263,586, US 4,295,133, disclose solutions related with this type of switch disposition.

Documents US 4,484,043 and US 6,696,653 B1 disclose a disposition of parallel conducting strips where there is a superposition between conductors that provided on a common vertical alignment along their extension.

Document EP 0671145 B1 discloses a similar disposition comprising a planar member underneath, a planar member over that deflects under the action of the weight of an individual and a sensor means placed between said planar members. This solution does not consider the measurement of variations of electrical conductivity nor provides an integration of a conducting textile disposition with a textile support.

Document US 6,987,232 B2 discloses a sensor and a method for detecting the movement of a patient, whereby the sensor uses a linearly configured resistive leader with resistors spaced apart as a means for determining at least approximately the location of a patient on the sensor, as well as an approximate length of the sensor that is compressed by the patient. The sensor comprises a top non-conducting member and a bottom non-conducting member.

Document US 7,396,331 B2 discloses a system and process for non-invasive collection and analysis of physiological signals.

Document WO 2008/104918 A1 discloses a system and method for obtaining physiologic data from a patient. Said system comprises at least one elastically deformable member, whereby said member is deformable by the patient lying in a bed. The method includes acquisition of deformation data of said member and the determination of physiological data of said patient based upon said deformation data.

Document WO 2009/124397 A2 discloses a signalling device for detecting the presence of an object, comprising first and second spacing elements positions in top and bottom layers, so that when forces are externally applied, the spacing elements collapse and there is established a conducting path between said first and second conductors.

Document WO 2013/003963 A1 discloses a device for detecting positional changes of people lying in beds. Said device comprises at least one elongated bridge for support of mattress and for transmission of the supported load. Said bridge comprises at least one plate with at least one section that is configured resistant to flexion. In the path of transmission of force between the load support and the support device there is disposed at least one sensor for detecting a measurement variable that can be influence by the supported load. This solution does not consider either measurement of variations of electric conductibility nor provides an integration of a conducting textile disposition with a textile support.

Document US 2017/042340 A1 refers to an occupancy detection arrangement for furniture such as a bed, by means of capacitance detection.

Document US 2012/156926 A1 discloses a connection member which is electrically connected to a conductive fabric having conductive threads, whereby the connection member includes a band-shaped part including conductive wires which extend in a longitudinal direction, and a connection terminal which is attached to an end portion of the conductive wires.

None of the documents in prior art discloses an integration of several electrical components in a textile cover disposition adapted for being applied upon an object for supporting individuals, such as for example a mattress. There is therefore the need for a cover disposition of this type that provides more reliable and efficient electrical connections, as well as a quick and ergonomic installation, without the need for particular care with components and electrical connections.

The objective of the present invention is to provide a monitoring disposition for monitoring of an individual in a respective support surface, in particular on a support surface that comprises a textile material support, presenting more reliable and efficient electricity conducting connections between a conducting textile disposition and a respective control device.

In particular, it is an objective to provide technical conditions for a reliable measurement of at least one relevant electricity parameter of said conducting textiles, as result of solicitations, in particular forces generally along the direction of the gravity force, upon an associated textile material surface.

The objective above is solved according to the present invention by means of a monitoring disposition according to claim 1 and the invention is as defined in claim 1.

Preferred embodiments are defined in the dependent claims.

A related objective of the present invention is to provide a monitoring disposition that provides a simpler construction, a better integration of electric components and connections.

In particular, the present invention provides a more reliable and efficient association of a plurality of conducting textiles with a respective textile material support, and with an object adapted for supporting individuals, so as to provide a reliable measurement by the conducting textiles of solicitations upon said textile material support.

Moreover, said textile material support is provided so that can be applied upon said object so that said conducting textile disposition is substantially retained on said object, at least with relation to movements in a longitudinal extension of said object, and whereby said control device and the electrical connections between the latter and said conducting textile disposition are associated with said textile material support, preferentially retained thereon, and said electric connections are provided so that the placement of said textile material support upon said object does not require establishing any electric connection, and the removal of said textile material support does not require a disconnection of any electric connection.

It is herewith provided a textile cover disposition comprising several electrical means, adapted for direct and quick application, without great installation requirements and with smaller risks of bad functioning or low measurement reliability.

Said textile material support is provided as a cover element of at least one surface adapted for an individual to laydown, sit or layover, of an object for supporting individuals, including piece of furniture, such as for example chair, or support for accommodating individuals, such as for example the mattress of a bed.

A related objective of the present invention is to provide a monitoring disposition that provides a more reliable and efficient monitoring of the presence and movement of an individual on a respective object for supporting individuals, such as for example the mattress of a bed.

In particular, the monitoring disposition according to the present invention comprises a textile material support that presents a plurality of conducting textile wires, and at least one control device adapted so that it can apply an electric tension in successive moments, measuring at least one electric conductibility parameter, comparing with at least one previously measure value in each one of said conducting textile wires, so as to identify a variation of said electric conductibility parameter.

The present invention shall hereinafter be explained in greater detail based upon preferred embodiments and in the attached Figures.

The Figures show, in simplified schematic representations:
- Figure 1:: diagram of an embodiment of a monitoring unit (1) of a monitoring disposition;
- Figure 2:: diagram of a plurality of monitoring units (1) included in a monitoring disposition;
- Figure 3:: diagram of a conducting textile disposition (31) in a textile material support (2) in a monitoring disposition (1);
- Figure 4:: diagram of a conducting textile disposition (3) in a monitoring disposition (1);
- Figure 5:: diagram of a conducting textile disposition (3) of textile material support (2) in a monitoring disposition (1);
- Figure 6:: diagram of a conducting textile disposition (3) of textile material support (2) in a monitoring disposition (1) according to the invention;
- Figure 7:: diagram of a configuration of textile material support (2) in a monitoring disposition (1) according to the invention, in an open position;
- Figure 8:: diagram of a configuration of textile material support (2) in a monitoring disposition (1) according to the invention, in a closed position;
- Figure 9:: diagram of an electricity conducting disposition between a conducting textile disposition (3) and a respective control device (7) in a monitoring system according to the invention, with first connection means (4) open;
- Figure 10:: diagram of an electricity conducting disposition between a conducting textile disposition (3) and a respective control device (7) in a monitoring system according to the invention, with first connection means (4) closed;
- Figure 11:: assembly diagram of a monitoring disposition (1) not part of the present invention;
- Figure 12:: assembly diagram of the monitoring disposition (1) according to Figure 11;
- Figure 13:: assembly diagram of another monitoring disposition (1) not part of the present invention;
- Figure 14:: assembly diagram of the monitoring disposition (1) according to Figure 13;
- Figure 15:: Detail of assembly of the monitoring disposition (1) according to Figure 14;
- Figure 16:: diagram of the monitoring disposition (1) according to Figures 13 to 15.

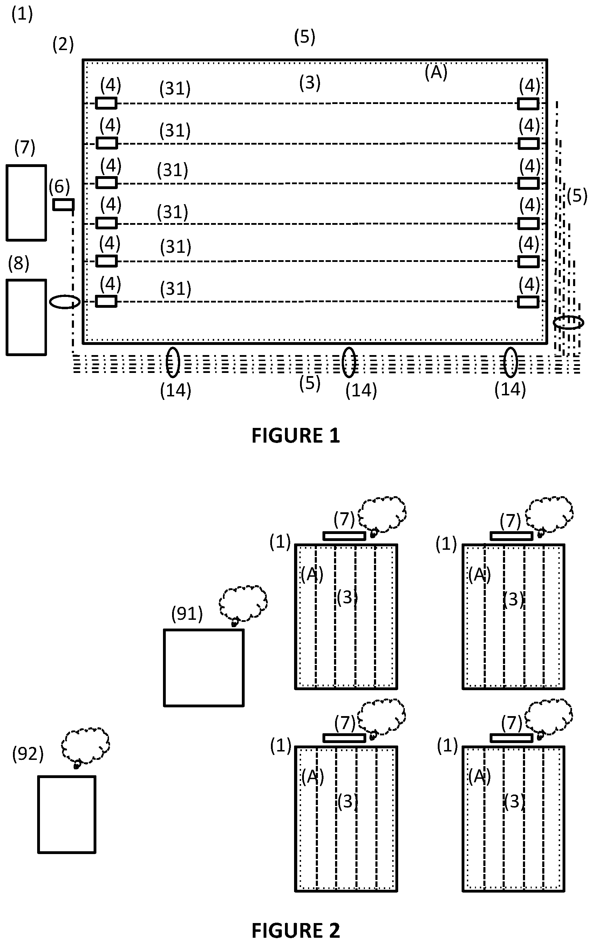

Figure 1 represents a preferred embodiment of a monitoring system, comprising a conducting textiles disposition (3) associated with a textile material support (2) and provided in conducting connection of electric energy with a control device (7).

The conducting textile disposition (3) comprises a plurality of conducting textiles (31) disposed along a substantially parallel direction relative to each other, and at a distance (D) between each other, whereby an electric tension can be applied between first and second ends of each one of said conducting textiles (31).

According to a first aspect, said conducting textiles (31) comprise a plurality of conducting particles provided along the elongated extension of said conducting textiles. Moreover, said conducting textiles (31) are adapted so that they provide a measurable variation of electric conductibility when submitted to stretching, including as a result of deflection along a direction transversal to respective elongated extension, in at least one region of respective elongated extension, so as to generate a variation of at least of distance and relative orientation between conducting particles provided on said conducting textiles (31) along the respective elongated extension.

In particular, said conducting textiles disposition (3) comprises a plurality of first conducting textiles (31) generally disposed along a first direction of said textile material support (2) and adapted so that they provide a measurable variation of electric conductibility when submitted to a force presenting a component perpendicular to the support surface provided by said textile material support (2).

Said conducting textiles (31) are provided as a textile material structure that presents a distribution of metallic particles provided with a given density and alignment so that provide electric energy conducting properties to said textile structure that vary according to the stretching of said conducting textiles (31) relative to a non-stretched condition, whereby said textile structure is preferentially of the type textile yarn, textile string, textile strip, or similar. The application of electric tension enable therefore measuring at least one parameter relating to the electric conductibility, for example the electric resistance, of each conducting textile (31), so that variations of said parameter in time provide an indication of load variations thereupon.

**Figure 2** represents a system comprising a plurality of monitoring units (1), for example beds, presenting respective control devices (7) provided in short range data connection, for example by means of Bluetooth, or similar, with a first remote data means (91) that can communicate data received with a second remote data means (92), for example by means of WIFI, or similar.

It is further preferred when the process of operation of a system further comprises the presentation of measured data, including data relating to types of events, respective time of occurrence, determined based upon periodic measurement of variations of at least one parameter of electric conductibility.

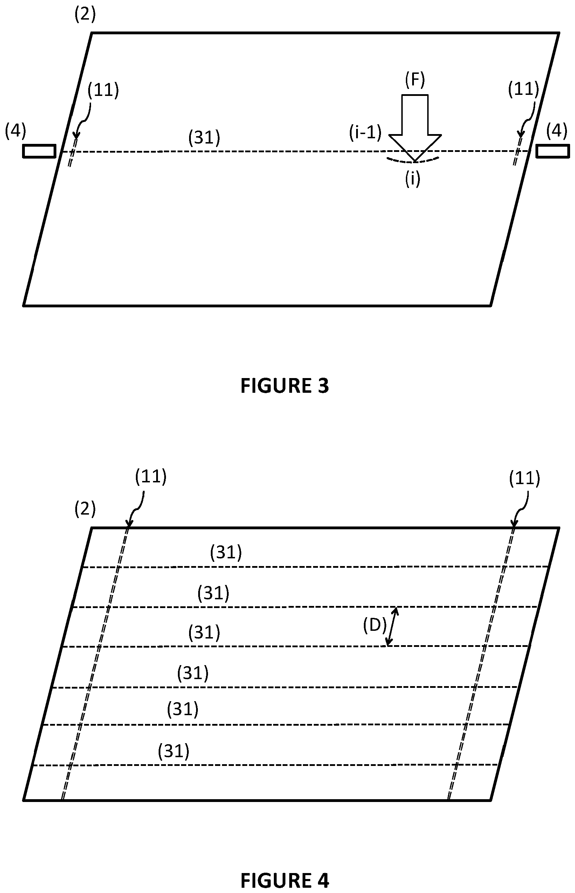

As schematically represented in **Figures 1** **and** **3****,** said control device (7) is associated to an energy source (8), and adapted so that can regulate by means of said control device (7), the realization of successive cycles i, i+1, ..., whereby each cycle comprises the application of a previously defined electric tension to each one of the conducting textiles (31) of said conducting textiles disposition (3), the measurement of at least one energy parameter related with the electric conductibility through said conducting textiles (31) and the assessment of an eventual variation in the measured value of said energy parameter with relation to at least one previous measurement.

Moreover, each monitoring cycle further includes the step of determining, based upon the value measured in a cycle i, a correspondence between at least one of:
- a variation previously measure in at least one cycle i-1 to i-n, and
- a plurality of previously measured variations in at least two cycles i-1 to i-n,
and a given type of event, including at least one of:
- entry and exit of an individual in contact with said conducting textile disposition (3);
- movement of an individual in contact with said conducting textile disposition (3).

According to a preferred embodiment, each monitoring cycle further includes determining a relative location on said conducting textile disposition (3), of a given interval of measured variations for at least one parameter associated with the electric conductibility of said conducting textile disposition (3).

Moreover, it is preferred when the control device (7) is provided so that can determine a relation between variations above a given threshold and the location of variations of at least one parameter associated to the electric conductibility on said conducting textile disposition (3), and respective evolution in time.

According to a preferred embodiment, said control device (7) is provided so that controls the application of an electric tension successively to the ends of each one of a plurality of conducting textiles (31), with a periodicity comprised between 0,001 s and 0,1 s, in ongoing manner in time.

According to a preferred embodiment (see **Figure 4**), both end regions of said conducting textiles (31) in the proximity of the limits of the top part are additionally fixed to the textile material support (2) by means of fixation means (11), provided so as to prevent the possibility of substantial displacement along the elongated extension thereof with relation to said textile material support (2). It is thereby ensured that the stretching of said conducting textiles (31) provides a reliable indication of the solicitation by a load applied upon said textile material support (2).

It is preferred when said conducting textiles are provided at distances (D) between each other of at least 10 mm, preferentially at least 20 mm, and at most 50 mm, preferentially at most 30 mm.

According to another aspect, said textile material support (2) is provided so that can be applied upon said object (A) for supporting of individuals, for example a mattress, so that said conducting textiles disposition (3) is substantially retained upon said object (A), at least with relation to movements along a longitudinal extension of said object (A). Moreover, said control device (7) and electric connections between the latter and said conducting textiles disposition (3) are associated with said textile material support (2), preferentially retained thereon, and said electric connections are provided so that the placement of said textile material support (2) on said object (A) does not require establishing any electric connection, and the removal of said textile material support (2) does not require disconnecting any electric connection.

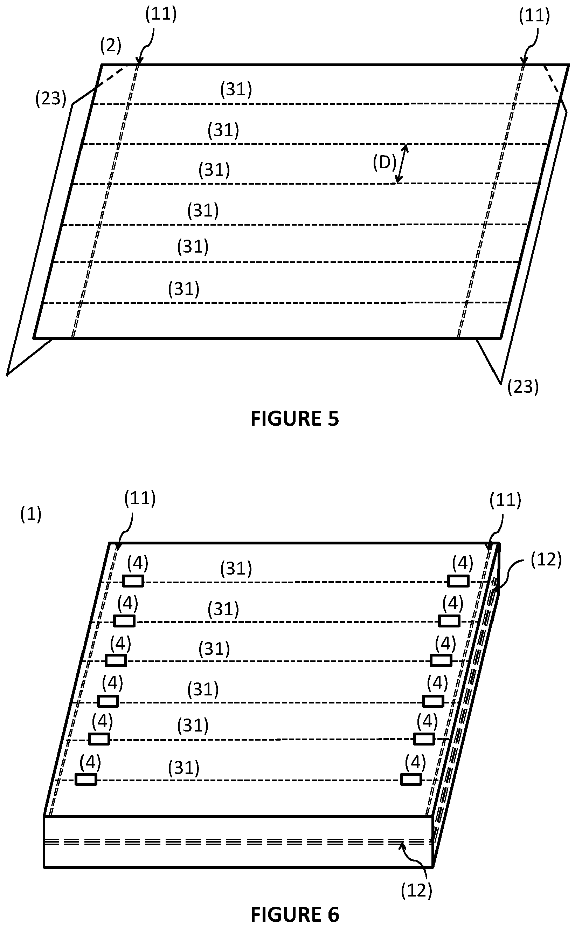

In the case of a first embodiment (1) represented in **Figure 5**, the textile material support (2) is provided with retention means (23) provided so that can retain said textile material support (2) in a substantially fixed position with relation to said object (A).

**Figure 6** represents an embodiment of a monitoring unit (1) according to the invention, where the textile material support (2) presents an at least partially closed general configuration so that can collect at least partially said object (A) inside thereof underneath said top part and in a substantially fixed position with relation to said textile material support (2).

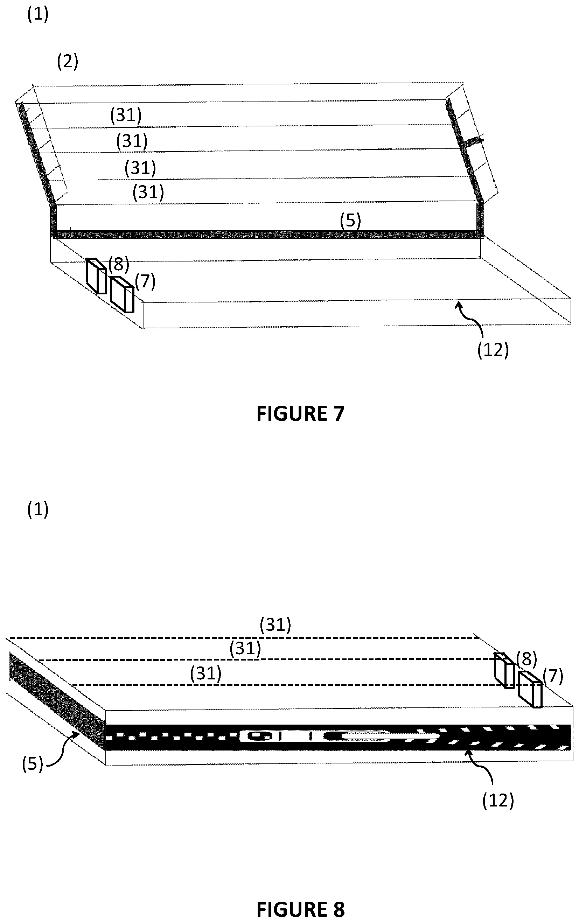

**Figures 7** and **8** represent a monitoring unit (1) corresponding to the embodiment according to the invention represented in Figure 6, with a general configuration of box type, or envelope, in positions of open and closed, respectively.

In particular, it is preferred when said textile material support (2) presents support connection means (12) adapted so as to provide connection between at least two of said parts of textile material support (2), such as for example of said top part (21, 22) to the remaining parts, along at least part of a perimeter region of textile material support (2), so that said textile material support (2) can be opened, applied as envelope of a support element, and closed, and vice-versa.

It is further according to the invention when said textile material support (2) is provided with a general shape so as to confine said object (A) for supporting individuals inside thereof, and when said control device (7), energy source (8) and all the energy conducting connections (4, 5, 6) between said conducting textile disposition (3) and said control device (7) are provided inside of the confinement provided by said textile material support (2), so that each monitoring unit (1) does not present wire connections to the exterior, for example to external devices.

This type of configuration of monitoring unit (1) provides a rapid application upon an object (A), such as for example a mattress, without the need to carry out an installation of electric components or of establishing electric connection between thereof.

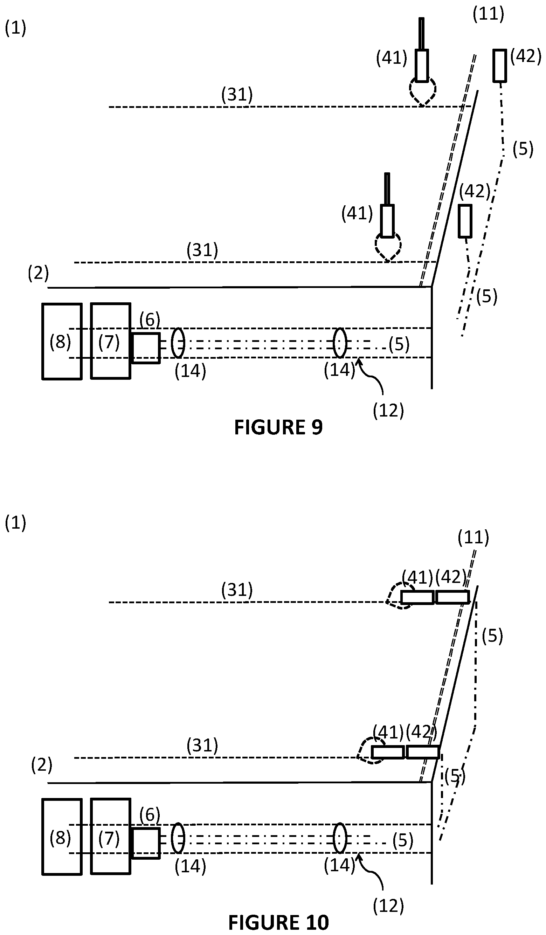

According to another inventive aspect (see **Figures 9** and 10), the energy conducting connection between said conducting textile disposition (3) and said control device (7), comprises first connection means (4) adapted for conducting connection of a first and second end of said conducting textile disposition (3), and energy conducting disposition (5) adapted for connection of said first connection means (4) and second connection means (6) adapted for conducting connection of said energy conducting disposition (5) to said control device (7), whereby both of said first and second connection means (4, 6) are provided as pieces comprising respective conducting metallic parts. Moreover, said first connection means (4) are adapted so that can be fixed to said conducting textiles (31) by means of a clamping type of connection.

It is further preferred when said conducting textiles (31) are united to respective conducting elements of said energy conducting disposition (5), by means of first connection means (4) provided in a region of said conducting textiles (31) next to said fixation means (11), preferentially respectively on the inward side with reference to the end of the top part of textile material support (2). As further represented in Figures 9 and 10, said first connection means (4) are advantageously provided as male-female type of connections (41, 42), presenting a part of conducting textile connection (31) by means of compression, and a conducting connection part. It is preferred when the removal of said connection by compression requires the use of a tool. It is preferred when said first connection means (4) result on the top part, next to an end thereof.

Moreover, it is preferred when said energy conducting disposition (5) passes along at least part of the extension of said textile material support and is retained along at least part of a lateral part by a plurality of retention elements (14) associated with said textile material support (2) and configured in the form of a link of a chain, or similar, so as to keep the plurality of conducting yarns included on said energy conduction disposition (5) in relative proximity between each other along at least part of respective extension.

It is preferred when said energy conducting disposition (5) is retained along at least part of its extension by a plurality of retention elements (14) associated with said textile material support (2) and configured in form of a link of a chain, or similar, so as to keep the plurality of conducting yarns included on said energy conducting disposition (5) in relative proximity between each other along at least part of respective extension.

An electric connection of simple construction, but reliable is herewith advantageously provided, in particular resistant to the movements by an individual on said top part of textile material support (2) that presents said conducting textile disposition (3).

In the case of another implementation not forming part of the present invention, the monitoring disposition (1) presents a textile material support (2) that comprises a first support element (21) presenting a conducting textile disposition (3) with first conducting textiles (31) developing along a first direction, and a second support element (22) presenting a textile disposition (3) with second conducting textiles (32) developing along a second direction orthogonal with relation to said first direction.

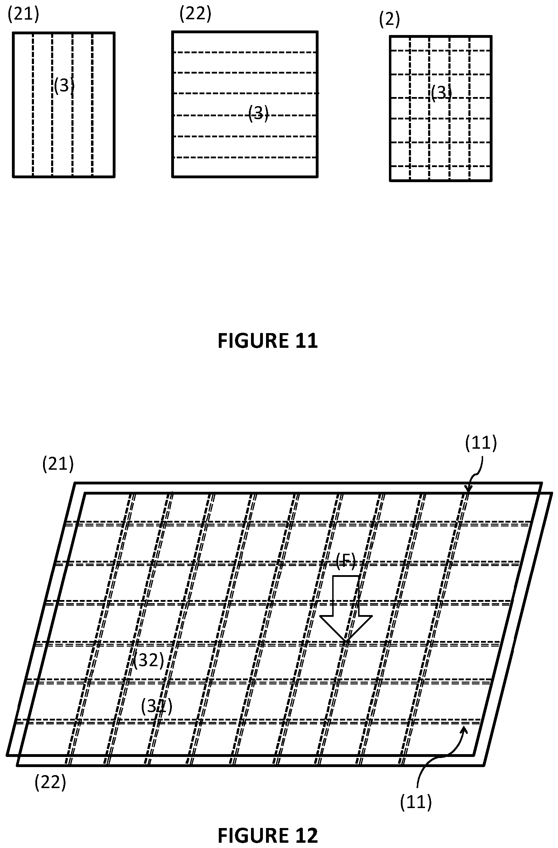

It is preferred when, as represented in **Figure 11****,** said first and second support elements (21, 22) are initially produced separately, preferentially by means of a similar process and machine, and associated with each other by means of superposition so that there results a layered disposition and with a matrix-like configuration of respective first and second conducting textiles (31, 32) .

As represented in **Figure 12****,** in the case there in an electric tension available at the ends of first and second conducting textiles (31, 32), the application of a force with a component transversal to the surface of the top part of said textile material support (2) thus results in the establishment of an electric contact in the intersecting zones.

As further represented, the conducting textiles (31, 32) are in this case retained in the textile material support (2) by fixing means (11) that develop along respective elongated extension. Moreover, said first connection means (4) would in this case be provided in the proximity of the retention surface - not represented -, as shall be promptly understood by the expert in the field.

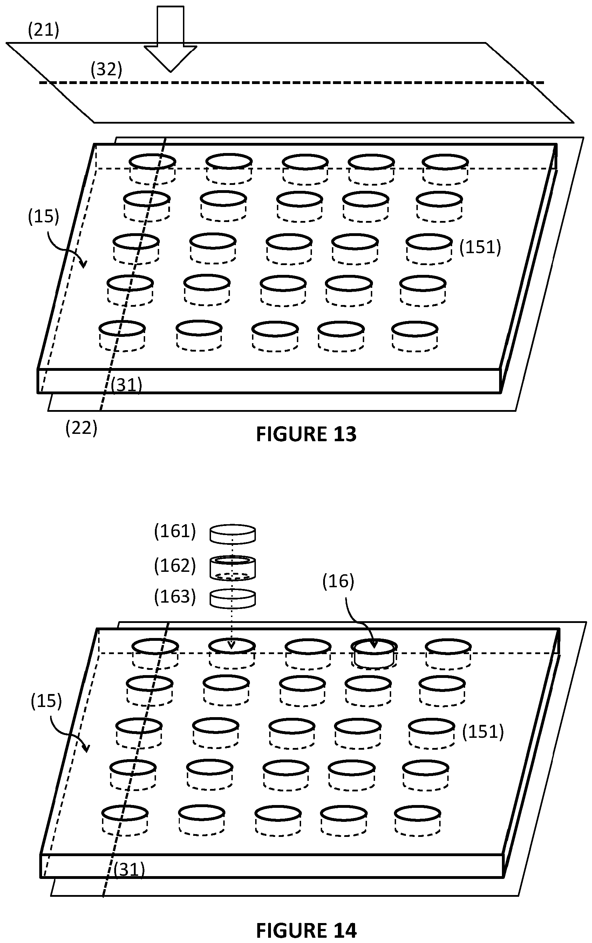

According to another implementation not forming part of the present invention, represented in **Figure** 13, the monitoring disposition (1) further presents and electric insulating element (15) arranged between first and second support elements (21, 22) of said textile material support (2), whereby said electric insulating element (15) presents a similar shape to the superposed top parts of said first and second support elements, there is provided an electric insulating material, such as for example a synthetic material, with a foam-like structure or similar, and presents a plurality of through-holes (151) provided in regions corresponding to the regions of intersection in superposition of said first and second conducting textiles (31, 32), so that provide and electric contact between these when said monitoring disposition (1) is submitted to a load long the direction of the gravity force.

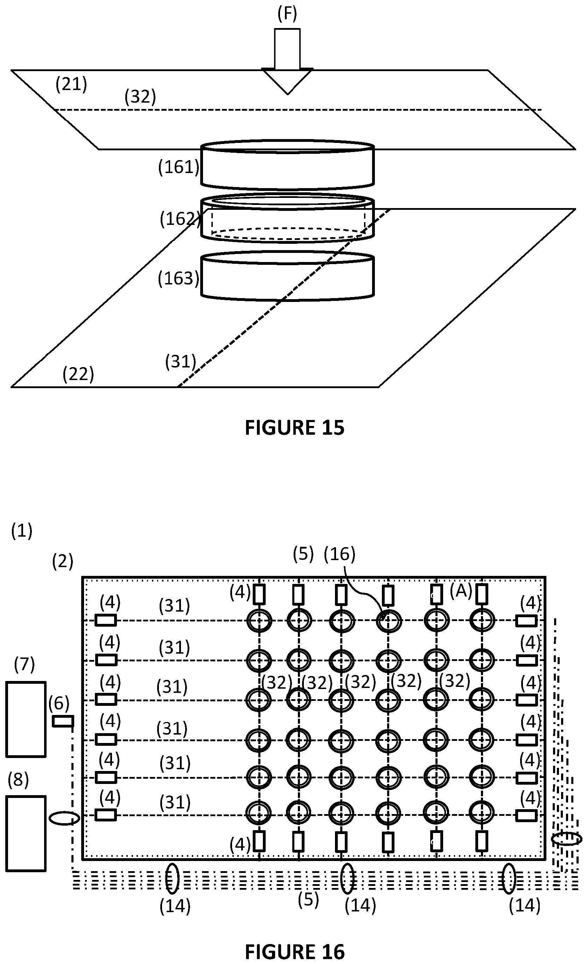

According to another aspect of the implementation not forming part of the present invention, represented in **Figures 14** and **15****,** the monitoring disposition (1) presents an electric insulation element (15) arranged between first and second support elements (21, 22) of said textile material support (2), presenting a plurality of through-holes (151), whereby each one of said through-holes (151) presents a pressure-switch disposition (16) that comprises a first element (161) in an electric conducting material, with a foam-like structure or similar, presenting a thickness of up to 5 mm, and a distancing element (162) provided in a non-conducting material, preferentially with a foam-like structure, configured for example with a ring-like shape of similar, presenting a height of up to 5 mm.

It is further preferred when said pressure switch disposition (16) comprises a second conducting foam layer (163), in electric conducting material, with a foam-like structure or similar, presenting a thickness of up to 5 mm. **Figure 16** shows another diagram of the set of said implementation not forming part of the present invention.

In the case of this embodiment, the control device (7) is adapted so that applies an electric tension to a first textile yarn (31) in a first direction, and measure the electric tensions available at that moment in each one of the textile yarns (2) along a second direction, repeating thereafter this cycle successively for each one of the remaining first textile yarns (31) along a first direction. As surely promptly understood by the expert in the technique, the application of a force upon the textile material support (2) shall produce a contact between said first textile yarn (31) and at least a second textile yarn (32), whereby said control device (7) is further adapted so that can reference the relative position of first and second textile yarns (31, 32) where there is measure an electric voltage resulting from contact between them.

The data measured by the control device (7) can be registered and related with other data measured in time, so as to establish relations with types of events and behaviour patterns.

## Claims

1. **Monitoring disposition (1)** for monitoring individuals in surfaces supporting individuals, whereby said monitoring disposition (1) comprises:
- a textile material support (2) presenting a top part (21, 22) adapted for covering at least part of the upwards-oriented surface of an object (A) that is adapted for support of individuals;
- a conducting textiles disposition (3) comprising a plurality of conducting textiles (31, 32) associated with said textile material support (2);
- a control device (7) provided in energy conducting connection with said conducting textile disposition (3) and with an energy source (8),
whereby said textile material support (2) presents a top part (21, 22) that comprises at least part of said conducting textile disposition (3),
whereby said textile material support (2) presents an at least partially closed general configuration of box type that can collect at least partially said object (A) inside thereof,
**characterized**
**in that** the energy conducting connection between said conducting textile disposition (3) and said control device (7), comprises:
- first connection means (4) adapted for conducting connection of a first or second ends of said conducting textiles disposition (3);
- an energy conducting disposition (5) adapted for connection of said first connection means (4) to second connection means (6);
- second connection means (6) adapted for conducting connection of said energy conduction disposition (5) to said control device (7),
whereby both of said first and second connection means (4, 6) are provided as pieces comprising respective conducting metallic part, and said first connection means (4) are adapted so that they can be fixed to said conducting textiles (31, 32) by means of a clamping/ compression type connection, and
**in that** said control device (7), energy source (8) and all the electric connections between said control device (7) and said conducting textiles disposition (3) are retained on said textile material support (2), and are provided inside of the confinement provided by said textile material support (2), so that the placement of said textile material support (2) upon said object (A) does not require establishing any electric connection, and the removal of said textile material support (2) does not require disconnecting any electric connection.

2. Disposition according to claim 1, **characterized in that** said conducting textiles (31, 32) are retained in respective end regions by fixation means (11), preferentially close the perimeter region of said top part of said textile material support (2), or along its entire elongated extension, so that they are retained at least partially against the possibility of displacement of said conducting textiles (31, 32) along the respective elongated extension.

3. Disposition according to claim 2, **characterized in that** said conducting textiles (31, 32) are connected with respective conducting elements of said energy conducting disposition (5), by first connection means (4) provided in a region of said conducting textiles (31, 32) next to the edge of the top part of the textile material support (2), preferentially next to said fixation means (11) of conducting textiles (31, 32).

4. Disposition according to claim 2 or 3, **characterized in that** said conducting textiles (31, 32) are connected with said conducting elements of said energy conduction disposition (5), by means of a portion of said conducting textiles (31, 32) that is raised above on a reduced extension with relation to the extension of said textile material support (2), in manner of a portion with the shape of a link of a chain preferentially next to said fixation means (11) of conducting textiles (31, 32).

5. Disposition according to any of previous claims 1 to 4, **characterized in that** said conducting textiles (31) are retained by fixation means (11) provided in the proximity of the edge of top part of said textile material support (2), preferentially by means of a seam onto said textile material support (2) along a transversal direction to the elongated extension of said conducting textiles (31), or **in that** said first and second conducting textiles (31, 32) are fixed to the textile material support (2) in a substantially continuous manner along respective elongated extension.

6. Disposition according to any of previous claims 1 to 5, **characterized in that** said control device (7) is provided on a lateral part of said textile material support (2) and in the proximity of an energy source (8), preferentially in the proximity of an opening region provided by support closing means (12).

7. Disposition according to any of previous claims 1 to 6, **characterized in that** said textile material support (2) presents support connection means (12) adapted so as to provide connection between at least two of said textile material support parts (2), and to provide a guiding support to at least part of the extension of said energy conduction disposition (5), whereby said guiding support is provided as at least one of a passageway in the shape of a link of a chain or a passageway in the shape of a sheath thereby containing said energy conduction disposition (5) in at least two regions, preferentially in a continuous extension, along at least part of a respective extension.

8. Disposition according to any of previous claims 1 to 7, **characterized in that** said energy conduction disposition (5) is retained along at least most part of its extension by a plurality of retention elements (14) associated with said textile material support (2) and configured in form of a sheath, or of a link of a chain, so as to keep the plurality of conducting yearns that are included in said energy conduction disposition (5) in relative proximity to each other along at least part of respective extension.

9. Disposition according to any of previous claims 1 to 8, **characterized in that** said textile material support (2) further presents retention means (23) provided so that they can retain said textile material support (2) in a substantially fixed position with relation to said object (A).

10. Disposition according to any of previous claims 1 to 10, **characterized in that** said textile material support (2) is provided with retention means (23) adapted so as to provide at least one of retention and fixation of said textile material support (2) on said object (A) for supporting individuals, so that said textile material support (2) can be placed and removed from said object (A) for support of individuals.

11. Disposition according to any of previous claims 1 to 11, **characterized in that** said textile material support (2) is provided with a general shape of a box, cylinder, sleeve, configured so that it can envelope at least most part of the exterior surface of an object (A) for supporting individuals, including of the type mattress, pillow, seat, and preferentially further at least one lateral part provided between said top part and base part, and a base part that does not present a conducting textile disposition (3).

12. Disposition according to any of previous claims, **characterized in that** said textile material support (2) presents support connection means (12) adapted so as to provide connection between at least two of said parts of textile material support (2), such as for example of said top part (21, 22) to the remaining parts, along at least part of a perimeter region of said textile material support (2).

13. Disposition according to any of previous claims, **characterized in that** said textile material support (2) presents support connection means (12) adapted so as to provide connection between at least two of said textile material support parts (2), of the type manually removable connection means, including of continuous form, such as for example of the zip type, and of punctual form, such as for example push button.

## Patentansprüche

1. Überwachungsanordnung (1) zur Überwachung von Personen in Oberflächen, die Personen tragen, wobei die Überwachungsanordnung (1) umfasst:
- einen Textilmaterialträger (2), der einen oberen Teil (21, 22) aufweist, der geeignet ist, mindestens einen Teil der nach oben gerichteten Oberfläche eines Objekts (A) zu bedecken, das zum Tragen von Personen geeignet ist;
- eine leitende Textilanordnung (3), die eine Vielzahl von leitenden Textilien (31, 32) umfasst, die mit dem Textilmaterialträger (2) verbunden sind;
- eine Steuervorrichtung (7), die in energieleitender Verbindung mit der leitenden Textilanordnung (3) und mit einer Energiequelle (8) steht,
wobei der Textilmaterialträger (2) einen oberen Teil (21, 22) aufweist, der zumindest einen Teil der leitenden Textilanordnung (3) umfasst,
wobei der Textilmaterialträger (2) eine zumindest teilweise geschlossene allgemeine Konfiguration des Kastentyps aufweist, die zumindest teilweise den Gegenstand (A) in ihrem Inneren aufnehmen kann, gekennzeichnet
dass die Energie leitende Verbindung zwischen der leitenden Textilanordnung (3) und der Steuervorrichtung (7) umfasst:
- ein erstes Verbindungsmittel (4), das für die leitende Verbindung eines ersten oder zweiten Endes der genannten leitenden Textilanordnung (3) geeignet ist;
- eine energieleitende Anordnung (5), die für die Verbindung des ersten Verbindungsmittels (4) mit dem zweiten Verbindungsmittel (6) geeignet ist;
- ein zweites Verbindungsmittel (6), das geeignet ist, die Verbindung zwischen der Energieübertragungsanordnung (5) und der Steuervorrichtung (7) herzustellen,
wobei sowohl das erste als auch das zweite Verbindungsmittel (4, 6) als Stücke vorgesehen sind, die jeweils ein leitendes Metallteil umfassen, und das erste Verbindungsmittel (4) so angepasst ist, dass es an den leitenden Textilien (31, 32) mittels einer Klemm-/Kompressionsverbindung befestigt werden kann, und
dass die Steuervorrichtung (7), die Energiequelle (8) und alle elektrischen Verbindungen zwischen der Steuervorrichtung (7) und der leitenden Textilanordnung (3) auf dem Textilmaterialträger (2) gehalten werden und innerhalb des von dem Textilmaterialträger (2) bereitgestellten Raums vorgesehen sind, so dass das Anbringen des Textilmaterialträgers (2) auf dem Objekt (A) keine Herstellung einer elektrischen Verbindung erfordert und das Entfernen des Textilmaterialträgers (2) kein Trennen einer elektrischen Verbindung erfordert.

2. Anordnung nach Anspruch 1, **dadurch gekennzeichnet, dass** die leitenden Textilien (31, 32) in den jeweiligen Endbereichen durch Befestigungsmittel (11) gehalten werden, vorzugsweise nahe dem Umfangsbereich des oberen Teils des Textilmaterialträgers (2) oder entlang seiner gesamten länglichen Ausdehnung, so dass sie zumindest teilweise gegen die Möglichkeit der Verschiebung der leitenden Textilien (31, 32) entlang der jeweiligen länglichen Ausdehnung gehalten werden.

3. Anordnung nach Anspruch 2, **dadurch gekennzeichnet, dass** die leitenden Textilien (31, 32) mit entsprechenden leitenden Elementen der energieleitenden Anordnung (5) durch erste Verbindungsmittel (4) verbunden sind, die in einem Bereich der leitenden Textilien (31, 32) nahe der Kante des oberen Teils des Textilmaterialträgers (2), vorzugsweise nahe den Befestigungsmitteln (11) der leitenden Textilien (31, 32), vorgesehen sind.

4. Anordnung nach Anspruch 2 oder 3, **dadurch gekennzeichnet, dass** die leitenden Textilien (31, 32) mit den leitenden Elementen der energieleitenden Anordnung (5) mittels eines Abschnitts der leitenden Textilien (31, 32) verbunden sind, der oben auf einer reduzierten Ausdehnung in Bezug auf die Ausdehnung des Textilmaterialträgers (2) angehoben ist, in der Art eines Abschnitts mit der Form eines Glieds einer Kette, vorzugsweise neben den Befestigungsmitteln (11) der leitenden Textilien (31, 32).

5. Anordnung nach einem der vorhergehenden Ansprüche 1 bis 4, **dadurch gekennzeichnet, dass** die leitenden Textilien (31) durch Befestigungsmittel (11) gehalten werden, die in der Nähe der Kante des oberen Teils des Textilmaterialträgers (2) vorgesehen sind, vorzugsweise mittels einer Naht an dem Textilmaterialträger (2) entlang einer Querrichtung zu der länglichen Ausdehnung der leitenden Textilien (31), oder dass die ersten und zweiten leitenden Textilien (31, 32) an dem Textilmaterialträger (2) in einer im Wesentlichen kontinuierlichen Weise entlang der jeweiligen länglichen Ausdehnung befestigt sind.

6. Anordnung nach einem der vorhergehenden Ansprüche 1 bis 5, **dadurch gekennzeichnet, dass** die Steuervorrichtung (7) an einem seitlichen Teil des Textilmaterialträgers (2) und in der Nähe einer Energiequelle (8) vorgesehen ist, vorzugsweise in der Nähe eines Öffnungsbereichs, der von einem Trägerverschlussmittel (12) bereitgestellt wird.

7. Anordnung nach einem der vorhergehenden Ansprüche 1 bis 6, **dadurch gekennzeichnet, dass** der Textilmaterialträger (2) Trägerverbindungsmittel (12) aufweist, die so angepasst sind, dass sie eine Verbindung zwischen mindestens zwei der Textilmaterialträgerteile (2) herstellen und eine Führungsunterstützung für mindestens einen Teil der Ausdehnung der energieleitenden Anordnung (5) bereitstellen, wobei die Führungsunterstützung durch mindestens eines von einem Durchgang in der Form eines Kettenglieds oder einem Durchgang in der Form einer Hülle gebildet ist, wodurch die energieleitende Anordnung (5) in mindestens zwei Bereichen, vorzugsweise in einer kontinuierlichen Ausdehnung, entlang mindestens eines Teils einer jeweiligen Ausdehnung enthalten ist.

8. Anordnung nach einem der vorhergehenden Ansprüche 1 bis 7, **dadurch gekennzeichnet, dass** die energieleitende Anordnung (5) entlang zumindest des größten Teils ihrer Ausdehnung durch eine Vielzahl von Rückhalteelementen (14) gehalten wird, die mit dem Textilmaterialträger (2) verbunden und in Form einer Hülle oder eines Kettenglieds konfiguriert sind, um die Vielzahl von leitenden Bändern, die in der energieleitenden Anordnung (5) enthalten sind, entlang zumindest eines Teils der jeweiligen Ausdehnung in relativer Nähe zueinander zu halten.

9. Anordnung nach einem der vorhergehenden Ansprüche 1 bis 8, **dadurch gekennzeichnet, dass** der Textilmaterialträger (2) ferner Haltemittel (23) aufweist, die so vorgesehen sind, dass sie den Textilmaterialträger (2) in einer im Wesentlichen festen Position in Bezug auf den Gegenstand (A) halten können.

10. Anordnung nach einem der vorhergehenden Ansprüche 1 bis 10, **dadurch gekennzeichnet, dass** der Textilmaterialträger (2) mit Haltemitteln (23) versehen ist, die so angepasst sind, dass sie mindestens eines von beiden, nämlich das Halten und die Fixierung des Textilmaterialträgers (2) auf dem Objekt (A) zur Unterstützung von Personen, gewährleisten, so dass der Textilmaterialträger (2) auf dem Objekt (A) zur Unterstützung von Personen platziert und von diesem entfernt werden kann.

11. Anordnung nach einem der vorhergehenden Ansprüche 1 bis 11, **dadurch gekennzeichnet, dass** der Träger (2) aus textilem Material die allgemeine Form eines Kastens, eines Zylinders, einer Hülse hat, die so gestaltet ist, dass sie zumindest den größten Teil der Außenfläche eines Objekts (A) zur Unterstützung von Personen, einschließlich des Typs Matratze, Kissen, Sitz, umhüllen kann, und vorzugsweise außerdem mindestens einen seitlichen Teil, der zwischen dem oberen Teil und dem Basisteil vorgesehen ist, und einen Basisteil aufweist, der keine leitende Textilanordnung (3) darstellt.

12. Anordnung nach einem der vorhergehenden Ansprüche, **dadurch gekennzeichnet, dass** der Textilmaterialträger (2) Trägerverbindungsmittel (12) aufweist, die so angepasst sind, dass sie eine Verbindung zwischen mindestens zwei der Teile des Textilmaterialträgers (2), wie zum Beispiel des oberen Teils (21, 22) mit den übrigen Teilen, entlang mindestens eines Teils eines Umfangsbereichs des Textilmaterialträgers (2) bereitstellen.

13. Anordnung nach einem der vorhergehenden Ansprüche, **dadurch gekennzeichnet, dass** der Träger (2) aus textilem Material Verbindungsmittel (12) aufweist, die geeignet sind, eine Verbindung zwischen mindestens zwei der Teile des Textilmaterialträgers (2) zu gewährleisten, und zwar von der Art manuell abnehmbarer Verbindungsmittel, einschließlich von kontinuierlicher Form, wie zum Beispiel vom Typ Reißverschluss, und von punktueller Form, wie zum Beispiel Druckknopf.

## Revendications

1. Dispositif de surveillance (1) pour surveiller des individus dans des surfaces supportant des individus, dans lequel ledit dispositif de surveillance (1) comprend :
- un support en matière textile (2) présentant une partie supérieure (21, 22) destinée à recouvrir au moins une partie de la surface orientée vers le haut d'un objet (A) destiné à supporter des individus ;
- une disposition de textiles conductrices (3) comprenant une pluralité de textiles conductrices (31, 32) associés audit support de matériau textile (2);
- un dispositif de commande (7) relié par conduction d'énergie à ladite disposition textile conductrice (3) et à une source d'énergie (8),
le support en matière textile (2) présente une partie supérieure (21, 22) qui comprend au moins une partie de la disposition textile conductrice (3),
ledit support en matière textile (2) présente une configuration générale au moins partiellement fermée de type boîte qui peut recueillir au moins partiellement ledit objet (A) à l'intérieur de celle-ci,
**caractérisé**
**en ce que** la connexion conductrice d'énergie entre ladite disposition textile conductrice (3) et ledit dispositif de commande (7), comprend :
- premier moyen de connexion (4) adapté à la connexion conductrice d'une première ou d'une deuxième extrémité de ladite disposition de textiles conductrices (3);
- une disposition conductrice d'énergie (5) adaptée à la connexion desdits premiers moyens de connexion (4) aux seconds moyens de connexion (6) ;
- deuxième moyen de connexion (6) adapté pour conduire la connexion de ladite disposition conductrice d'énergie (5) audit dispositif de contrôle (7),
lesdits premier et deuxième moyens de connexion (4, 6) sont fournis sous forme de pièces comprenant une partie métallique conductrice respective, et lesdits premiers moyens de connexion (4) sont adaptés de manière à pouvoir être fixés aux textiles conducteurs (31, 32) au moyen d'une connexion de type serrage/contrainte, et
**en ce que** ledit dispositif de commande (7), la source d'énergie (8) et toutes les connexions électriques entre ledit dispositif de commande (7) et ladite disposition de textiles conductrices (3) sont retenus sur ledit support en matière textile (2), et sont fournis à l'intérieur du confinement fourni par ledit support en matière textile (2), de sorte que le placement dudit support en matière textile (2) sur ledit objet (A) ne nécessite pas l'établissement d'une connexion électrique, et que le retrait dudit support en matière textile (2) ne nécessite pas la déconnexion d'une quelconque connexion électrique.

2. Disposition selon la revendication 1, **caractérisée en ce que** lesdits textiles conductrices (31, 32) sont retenus dans des régions d'extrémité respectives par des moyens de fixation (11), de préférence à proximité de la région périmétrique de ladite partie supérieure dudit support en matière textile (2), ou le long de toute son extension allongée, de sorte qu'ils sont retenus au moins partiellement contre la possibilité de déplacement desdits textiles conductrices (31, 32) le long de l'extension allongée respective.

3. Disposition selon la revendication 2, **caractérisée en ce que** lesdits textiles conducteurs (31, 32) sont reliés aux éléments conducteurs respectifs de ladite disposition conductrice d'énergie (5), par des premiers moyens de connexion (4) prévus dans une région desdits textiles conductrices (31, 32) à côté du bord de la partie supérieure du support en matière textile (2), de préférence à côté desdits moyens de fixation (11) des textiles conductrices (31, 32).

4. Disposition selon la revendication 2 ou 3, **caractérisée en ce que** lesdits textiles conductrices (31, 32) sont reliés auxdits éléments conducteurs de ladite disposition conductrice d'énergie (5), au moyen d'une partie desdits textiles conductrices (31, 32) qui est soulevée au-dessus sur une extension réduite par rapport à l'extension dudit support en matière textile (2), à la manière d'une partie ayant la forme d'un maillon de chaîne, de préférence à côté desdits moyens de fixation (11) des textiles conductrices (31, 32).

5. Disposition selon l'une quelconque des revendications précédentes 1 à 4, **caractérisée en ce que** lesdits textiles conductrices (31) sont retenus par des moyens de fixation (11) prévus à proximité du bord de la partie supérieure dudit support en matière textile (2), de préférence au moyen d'une couture sur ledit support en matière textile (2) le long d'une direction transversale à l'extension allongée desdits textiles conductrices (31), ou **en ce que** lesdits premier et second textiles conductrices (31, 32) sont fixés au support en matière textile (2) d'une manière sensiblement continue le long de l'extension allongée respective.

6. Disposition selon l'une quelconque des revendications précédentes 1 à 5, **caractérisée en ce que** ledit dispositif de contrôle (7) est prévu sur une partie latérale dudit support en matière textile (2) et à proximité d'une source d'énergie (8), préférentiellement à proximité d'une zone d'ouverture prévue par les moyens de fermeture du support (12).

7. Disposition selon l'une quelconque des revendications précédentes 1 à 6, **caractérisée en ce que** ledit support en matière textile (2) présente des moyens de connexion de support (12) adaptés pour assurer la connexion entre au moins deux desdites parties de support en matière textile (2), et pour fournir un support de guidage à au moins une partie de l'extension de ladite disposition conductrice d'énergie (5), ledit support de guidage est fourni sous la forme d'au moins un passage en forme de maillon de chaîne ou d'un passage en forme de gaine, contenant ainsi ladite disposition conductrice d'énergie (5) dans au moins deux régions, de préférence dans une extension continue, le long d'au moins une partie d'une extension respective.

8. Disposition selon l'une quelconque des revendications précédentes 1 à 7, **caractérisée en ce que** ladite disposition conductrice d'énergie (5) est retenue le long d'au moins la majeure partie de son extension par une pluralité d'éléments de rétention (14) associés audit support en matière textile (2) et configurés sous la forme d'une gaine ou d'un maillon d'une chaîne, de manière à maintenir la pluralité de fils conducteurs qui sont inclus dans ladite disposition conductrice d'énergie (5) à proximité relative les uns des autres le long d'au moins une partie de l'extension respective.

9. Disposition selon l'une quelconque des revendications précédentes 1 à 8, **caractérisée en ce que** ledit support en matière textile (2) présente en outre des moyens de rétention (23) prévus pour pouvoir retenir ledit support en matière textile (2) dans une position sensiblement fixe par rapport audit objet (A).

10. Disposition selon l'une quelconque des revendications précédentes 1 à 10, **caractérisée en ce que** ledit support en matière textile (2) est pourvu de moyens de rétention (23) adaptés de manière à assurer au moins l'une des fonctions de rétention et de fixation dudit support en matière textile (2) sur ledit objet (A) de support d'individus, de sorte que ledit support de matière textile (2) puisse être placé et retiré dudit objet (A) de support d'individus.

11. Disposition selon l'une quelconque des revendications précédentes 1 à 11, **caractérisée en ce que** ledit support en matière textile (2) est pourvu d'une forme générale de boîte, cylindre, manchon, configuré de manière à pouvoir envelopper au moins la majeure partie de la surface extérieure d'un objet (A) de soutien d'individus, y compris de type matelas, oreiller, siège, et préférentiellement en outre au moins une partie latérale prévue entre ladite partie supérieure et la partie de base, et une partie de base qui ne présente pas de disposition textile conductrice (3).

12. Disposition selon l'une quelconque des revendications précédentes, **caractérisée en ce que** ledit support en matière textile (2) présente des moyens de connexion de support (12) adaptés de manière à assurer la connexion entre au moins deux desdites parties du support en matière textile (2), comme par exemple de ladite partie supérieure (21, 22) aux parties restantes, le long d'au moins une partie d'une région périmétrique dudit support en matière textile (2).

13. Disposition selon l'une quelconque des revendications précédentes, **caractérisée en ce que** ledit support en matière textile (2) présente des moyens de liaison au support (12) adaptés de manière à assurer la liaison entre au moins deux desdites parties du support en matière textile (2), du type moyens de liaison amovibles manuellement, y compris de forme continue, telle que par exemple du type fermeture à glissière, et de forme ponctuelle, telle que par exemple bouton poussoir.
